Europäisches Patentamt
European Patent Office
Office européen des brevets

(11) Publication number: 0 259 990 A1

# EUROPEAN PATENT APPLICATION

(21) Application number: 87307295.3

(22) Date of filing: 18.08.87

(51) Int. Cl.4: A61K 33/00 , A61K 31/19 , //(A61K33/00,31:19),(A61K31/19,31:13)

(30) Priority: 25.08.86 US 900201

(43) Date of publication of application: 16.03.88 Bulletin 88/11

(84) Designated Contracting States: AT BE CH DE ES FR GB GR IT LI LU NL SE

(71) Applicant: MERCK & CO. INC.
126, East Lincoln Avenue P.O. Box 2000
Rahway New Jersey 07065-0900(US)

(72) Inventor: O'Neill, Joseph
403 Spruce Circle Bethel Grant
Lansdale Pennsylvania 19446(US)
Inventor: Dehner, Eugene J.
645 Franklin Street
Lansdale Pennsylvania 19446(US)

(74) Representative: Hesketh, Alan, Dr.
European Patent Department Merck & Co., Inc. Terlings Park Eastwick Road
Harlow Essex, CM20 2QR(GB)

## (54) Rapid acting combination of sodium sulindac and a base.

(57) A combination of sulindac or sodium sulindac and an appropriate base in excess and of a sufficient amount to neutralize gastric acidity for example, tromethamine or sodium bicarbonate, have been found to exhibit unexpectedly rapid absorption and improved onset of activity relative to sulindac and conventional water-soluble formulations containing sodium sulindac.

EP 0 259 990 A1

# RAPID ABSORBING AND ACTING COMBINATION OF SULINDAC OR SODIUM SULINDAC AND A BASE

## BACKGROUND OF THE INVENTION

It is well known that in evaluating a drug's merit, a faster onset of activity is one of the important considerations of the drug's overall therapeutic profile. An early onset is important. It brings the relief quickly and therefore promotes patient compliance.

Sulindac is a non-steroidal antiinflammatory and analgesic drug (NSAID) known since the early 1970's. It is claimed in U.S. Patent 3,654,349 issued to T. Y. Shen et al. in April 1972, and commercialized by Merck & Co., Inc. under the tradename CLINORIL. However, sulindac has a slow onset of activity which is due to its poor solubility in an acidic environment such as the stomach.

Sulindac is insoluble in water below pH 4.5, but its sodium salt is very soluble. It was thought that sodium sulindac, like sodium salts of other NSAIDs, for example, indomethacin, should have a faster bioavailability and a much shorter onset of activity than the parent compound, sulindac itself. Surprisingly, no appreciable difference in onsets was found between sulindac tablets and the aqueous solution of sodium sulindac in spite of numerous experiments. Based on historical data in man, sodium sulindac given as an oral solution exhibited no improved onset of activity when compared to sulindac given as a tablet, 10 mg/ml or 40 mg/ml oral suspension, a rectal suppository, an intramuscular injection suspension or even as its active metabolite (sulindac sulfide).

Tromethamine or sodium salts of indomethacin, naproxen and aspirin are known (British Patent 2,059,768; and German Patent 134,672). These salts as disclosed by these patents, show enhanced bioavailability of the drug. Thus, it was totally unexpected that no such conventional improvement of the rapidity of bioavailability or onset of activity was observed for the sodium sulindac and sulindac tromethamine salt when they were tested by themselves.

Although sodium sulindac, sodium bicarbonate and tromethamine are known, there is no known combination of these salts.

However, when a sufficient amount of a base such as tromethamine and/or sodium bicarbonate is combined with sodium sulindac, an unexpected improvement of bioavailability and an earlier onset of activity resulted.

It is therefore the object of this invention to provide a novel combination of sulindac or sodium sulindac and a base with a faster absorption and onset of activity, shorter time to peak activity and enhanced bioavailability than sulindac or sodium sulindac alone.

It is also the object of this invention to provide a pharmaceutical composition of the combination for the treatment of inflammation and pain.

Finally, it is the object of this invention to develop a method of treatment comprising the administration of the novel combination to patients suffering inflammation and pain.

## DETAILED DESCRIPTION OF THE INVENTION

The present invention is directed to a combination of sulindac or sodium sulindac and a base which may be tromethamine or sodium bicarbonate or a combination thereof. The base is present in an excess amount that will maintain the drug solubilized in an acidic environment such as the stomach and thus improve the absorption and shorten the time to peak activity by solubilizing and delivering sulindac from the stomach into the duodenum for effective and rapid absorption by the body system.

Sulindac is represented by the formula (A):

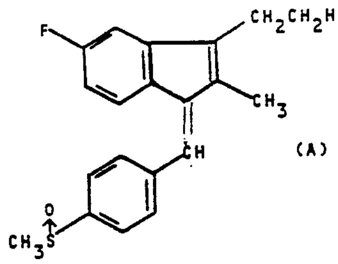

(A)

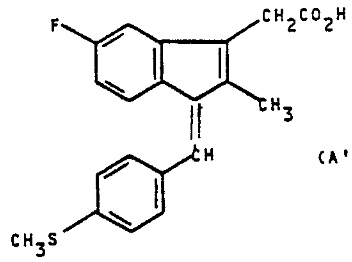

(A')

It is a NSAID with a long duration of action and is a reduction-oxidation prodrug which owes its in vivo activity to its metabolite, the active sulfide of formula (A').

Tromethamine is represented by the formula (B): $NH_2C(CH_2OH)_3$; and sodium bicarbonate is of formula: $NaHCO_3$.

The combination is used in an amount sufficient to treat inflammation and pain. Normally, the combination will contain the active ingredient, namely, sulindac or sodium sulindac in an amount of from about 2 mg to 20 mg per kg body weight per day (about l00 mg to lg per patient per day), for example, the therapeutic effect is obtained from oral administration of a daily dosage of from about 200 to 600 mg/day. It should be understood, however, that although dosage ranges are given, the dose level for any particular patient depends upon the age, body weight, sex, time of administration, route of administration, rate of excretion, drug combination, reaction sensitivities and severity of the particular disease of the patient.

For combinations containing sodium sulindac or sulindac and tromethamine, the weight ratio is about 25 mg to l000 mg of tromethamine to about 25 mg to 750 mg of sulindac or sodium sulindac. Preferably, 75 to 750 mg tromethamine is mixed with 50-450 mg of sulindac or sodium sulindac. Even more preferably, l50 to 700 mg of tromethamine is combined with 2l2 to 424 mg of sodium sulindac (corresponding to 200 to 400 mg free acid of sulindac). It should be noted that compressed tablets containing sodium sulindac and tromethamine do not disintegrate or dissolve rapidly at simulated gastric pH's. The addition of 0.5% to l6% of the tablet weight of some commonly used disintegrants or super disintegrants, e.g., Ac-Di-Sol® and Explotab® , failed to effect rapid disintegration and dissolution in the presence of the commonly used lubricant magnesium stearate.

To improve the rate of disintegration and dissolution, the combination comprises, in addition to tromethamine and sodium sulindac, about 2-l0% by weight of glyceryl behenate. For even more improved results, about 2-l0% by weight corn starch and/or about 0.l-0.5% by weight docusate sodium are added. The following is a representative combination of sodium sulindac and tromethamine.

| | mg/Tablet |
|---|---|
| Sodium Sulindac | 424 |
| Tromethamine | 295 or 300 |
| Docusate Sodium | 2 |
| Corn Starch | 30-60 |
| Glyceryl Behenate | 35-70 |

For combinations containing sodium sulindac and sodium bicarbonate, the quantity of sodium bicarbonate in weight is at about 0.8 g to 2.0 g which is the amount required to neutralize the stomach and thereby allowing the discharge of sodium sulindac from the stomach to the small intestine as a solution.

The following is a representative combination of sodium sulindac and sodium bicarbonate:

sodium sulindac - 0.2l2 g (equivalent to 200 mg of sulindac)

sodium bicarbonate - l.460 g

Although the preferred amount of sodium sulindac is about 0.2l2 g to 0.424 g, it can range from 0.l06 g to 0.636 g.

This invention also relates to a method of treating inflammation and pain in patients using the combination. For example, the combination of the invention can be used to treat diseases such as rheumatoid arthritis, osteoarthritis, gout, infectious arthritis and rheumatic fever. It has faster and better bioavailability, shorter onset of activity, shorter time to peak activity than sulindac or sodium sulindac alone and exhibits a lower incidence of vomiting and other adverse side effects than the parent compound itself.

The treatment of inflammation and pain can be accomplished by orally administering to patients a therapeutic dose of the combination, particularly in a non-toxic pharmaceutically acceptable carrier, preferably in tablet or solution form.

The non-toxic pharmaceutical carrier may be for example, either a solid or a liquid. Exemplary of solid carriers are lactose, corn starch, gelatin, talc, sterotex, glyceryl behenate, terra alba, docusote sodium, sucrose, agar, pectin, cab-o-sil, and acacia.

Exemplary of liquid carriers are peanut oil, olive oil, sesame oil and water, preferably water.

The combinations may be prepared by blending solindac or sodium sulindac, a base and a suitable binding agent in a suitable mixer and granulated by addition of alcohol such as anhydrous SD3A Alcohol. The wet mass is screened milled, dried, screened again and blended with disintegrants and lubricants before compression.

The following Examples illustrate the dissolution profiles of the combinations:

EXAMPLE I

The composition for 2l2 mg of sulindac sodium with tromethamine (TRIS) contained 70 mg glyceryl behenate, 2 mg docusate sodium and 60 mg corn starch. The formulation has the following dissolution profiles (USP at 50 RPM) in 900 ml 0.6 m Eq and 2.45 m Eq HCl Media.

% Dissolution in HCl Media

| Time (Minutes) | 212 mg sodium sulindac and 660 mg TRIS | | 212 mg sodium sulindac and 295 mg TRIS | |
|---|---|---|---|---|
| | 0.6 mEq HCl | 2.45 mEq HCl | 0.6 mEq HCl | 2.45 mEq HCl |
| 5 | 46 | 47 | — | — |
| 10 | 83 | 80 | 94 | 89 |
| 15 | 100 | 99 | — | — |
| 20 | 102 | 100 | 98 | 92 |
| 30 | 102 | 101 | 99 | 92 |

EXAMPLE 2

The. effect of glyceryl behenate, docusate sodium and/or corn starch to the combination of sulindac sodium (424 mg) and tromethamine (295 mg) is illustrated in the following table:

% Dissolution in 2.45 m Eq HCl Media

| Time (Minutes)/Combination | A[a] | B[b] | C[c] | D[d] |
|---|---|---|---|---|
| 10 | 8 | 12 | 70 | 89 |
| 20 | 49 | 80 | 97 | 92 |
| 30 | 88 | 82 | 96 | 92 |

a. A = sodium sulindac (424 mg)/Tromethamine (295 mg)
b. B = sodium sulindac (424 mg)/Tromethamine (295 mg) + 4% by weight glyceryl behenate
c. C = sodium sulindac (424 mg)Tromethamine (295 mg) + 4% by weight glyceryl behenate + 0.2% by weight docusate sodium
d. D = sulindac (424 mg)/tromethamine (295 mg) + 4% by weight glyceryl behenate + 0.2% by weight docusate + 5% corn starch.

Example 3

This was an open-label, single-dose, two-way crossover study in l8 healthy subjects. Sixteen completed the following treatments. Treatment days were separated by at least 7 days.

Treatment A: One CLINORIL (manufactured by Merck & Co. Inc.) tablet containing 400 mg sulindac

Treatment B: One sodium sulindac-tromethamine tablet containing 424 mg sodium sulindac and 295 mg tromethamine

Treatment C: One sodium sulindac-tromethamine tablet containing 424 mg sulindac and 660 mg tromethamine

Treatment D: Solution containing 424 mg sodium sulindac and 660 mg tromethamine

Plasma samples were collected at 0, 0.25, 0.5, 0.75, l, l.5, 2, 3, 4, and 6 hours. Complete urine samples were collected for the following intervals: -l to 0 hour (control), 0-2, 2-4, 4-6, 6-24, 24-48, 48-72, and 72-96 hours. All samples were kept frozen until the time of analysis.

Table l lists the physicochemical properties of the two dosage forms. Tables 2 summarizes the pharmacokinetic parameters. Based on the date shown, it is apparent that the total urinary recovery of sulindac are comparable between the four treatments. However, the time-to-plasma peak indicates an improvement in the absorption level and rate. The extent of absorption, as judged by the total urinery recoveries, was not affected.

Table I

Compositions of the Test Dosage Forms

| Ingredient | CLINORIL Tablet 400 mg | Na-sulindac Tromethamine Tablet 424/295 mg | Na-sulindac Tromethamine Tablet 424/660 mg | Solution of Clinoril Sod. Tromethamine 424/660 mg |
|---|---|---|---|---|
| | mg | mg | mg | mg |
| Sulindac | 400 | — | — | — |
| Sulindac Sodium | — | 424 | 424 | 424 |
| Tromethamine | — | 295 | 660 | 660 |
| Pregel. Starch | 20 | — | — | — |
| Microcryst. Cellulose | 245.6 | — | — | — |
| Mag. Stearate | 4.6 | — | — | — |
| Glyceryl Behemate | — | 30 | 70 | — |
| Corn Starch | — | 40 | 60 | — |
| Docusate Sodium | — | 1.5 | 2 | — |
| Aspartame | — | — | — | qs |
| Blood Orange | — | — | — | qs |
| Tablet Weight | 670 | 791 | 1216 | — |

Table 2. Observed Maximum Plasma Concentrations ($C_{max}$, mg/ml) for Sulindac and Metabolites

| | Treatment A | | | Treatment B | | | Treatment C | | | Treatment D | | |
|---|---|---|---|---|---|---|---|---|---|---|---|---|
| Subject | Sulindac | Sul-fone | Sul-fide | Sulindac | Sul-fone | Sul-fide | Sulindac | Sul-fone | Sul-fide | Sulindac | Sul-fone | Sul-fide |
| 1 | 4.5 | 1.6 | 4.4 | 18.2 | 2.1 | 7.0 | 20.4 | 1.3 | 5.3 | 20.6 | 2.3 | 5.8 |
| 2 | 12.8 | 1.8 | 5.4 | 8.7 | 1.9 | 4.0 | 19.3 | 2.1 | 5.1 | 16.0 | 2.1 | 5.7 |
| 3 | 10.3 | 2.8 | 6.5 | 7.3 | 8.3 | 5.3 | 15.7 | 2.4 | 6.1 | 20.2 | 2.3 | 6.4 |
| 4 | 11.8 | 2.0 | 5.5 | 20.9 | 1.9 | 5.7 | 26.6 | 2.0[a] | 7.0[a] | 8.5[a] | 2.5 | 5.0 |
| 5 | 1.9 | 0.5 | 0.5 | 13.1 | 3.0 | 6.9 | 19.5 | 1.5 | 5.4 | 19.5 | 1.9 | 8.1 |
| 6 | 7.1 | 2.9 | 4.7 | 21.4 | 3.2 | 7.4 | 22.2 | 16.7 | 11.0 | 26.8 | 3.6[a] | 9.5 |
| 7 | 12.8 | 2.1 | 7.2 | 29.6 | 1.8[a] | 8.1 | 24.3 | 2.8 | 8.8 | 15.8 | 1.8[a] | 10.7 |
| 8 | 13.3 | 1.6 | 6.2 | 29.7 | 2.1 | 7.6 | 26.0 | 1.7 | 6.5[a] | 32.7 | 2.3 | 9.8 |
| 9 | 5.0 | 2.9 | 2.7 | 19.0 | 2.4 | 2.9 | 6.5 | 2.4 | 2.2 | 17.9 | 4.0 | 5.4 |
| 10 | 8.4 | 2.2 | 4.9 | 11.8 | 1.7 | 4.5[a] | 27.5 | 2.2 | 6.8 | 20.2 | 2.4 | 7.7 |
| 11 | 2.5 | 1.6 | 1.5 | 3.0 | 1.4 | 1.2 | 6.9 | 2.0 | 1.8 | 10.8 | 1.8 | 2.3 |
| 12 | 5.0 | 2.9 | 2.8 | 12.5 | 2.3 | 4.5[a] | 15.0 | 2.3 | 3.7 | 13.8 | 2.1 | 2.9 |
| 13 | 20.4 | 3.2 | 9.3 | 37.9 | 3.1 | 8.9 | 30.4 | 2.1 | 6.8 | 33.3 | 2.2 | 5.2 |
| 14 | 21.5 | 3.9 | 10.3[a] | 38.0 | 3.0 | 9.9 | 37.6 | 2.5 | 9.6 | 20.4 | 1.9[a] | 5.6[a] |
| 15 | 8.4 | 2.9 | 6.2 | 29.2 | 2.8[a] | 8.2 | 22.9 | 3.0 | 8.3 | 19.9 | 2.4[a] | 7.6[a] |
| 16 | 8.6 | 2.5 | 4.5 | 16.4 | 2.3 | 5.0 | 24.1 | 2.3 | 4.8 | 26.3 | 2.2 | 5.8 |
| Mean | 9.6 | 2.3 | 5.2 | 19.8 | 2.7 | 6.1 | 21.6 | 3.1 | 6.2 | 20.2 | 2.4 | 6.5 |
| S.D. | 5.7 | 0.8 | 2.6 | 10.6 | 1.6 | 2.3 | 8.0 | 3.7 | 2.5 | 6.9 | 0.6 | 2.3 |

Treatment A - 400-mg tablet CLINORIL.
Treatment B - Tablet containing 424 mg sodium sulindac and 296 mg tromethamine.
Treatment C - Tablet containing 424 mg sodium sulindac and 660 mg tromethamine.
Treatment D - Solution containing 424 mg sodium sulindac and 660 mg tromethamine.
[a] - Evidence of enterohepatic recycling - value corresponding to earliest $t_{max}$ value chosen.

Table 3. Times ($t_{max}$, min.) At Which Maximum Plasma Concentrations Occur for Sulindac and Metabolites

| | Treatment A | | | Treatment B | | | Treatment C | | | Treatment D | | |
|---|---|---|---|---|---|---|---|---|---|---|---|---|
| Subject | Sulindac | Sul-fone | Sul-fide | Sulindac | Sul-fone | Sul-fide | Sulindac | Sul-fone | Sul-fide | Sulindac | Sul-fone | Sul-fide |
| 1 | 60 | 240 | 240 | 30 | 120 | 90 | 30 | 60 | 90 | 20 | 60 | 90 |
| 2 | 60 | 120 | 150 | 30 | 90 | 150 | 40 | 90 | 120 | 30 | 90 | 90 |
| 3 | 60 | 120 | 150 | 20 | 20 | 240 | 30 | 60 | 60 | 20 | 90 | 90 |
| 4 | 90 | 240 | 240 | 30 | 90 | 90 | 60 | 90[a] | 120[a] | 40[a] | 120 | 120 |
| 5 | 240 | 240 | 240 | 30 | 120 | 120 | 30 | 90 | 90 | 20 | 60 | 90 |
| 6 | 120 | 240 | 240 | 30 | 90 | 90 | 30 | 60 | 60 | 20 | 40[a] | 120 |
| 7 | 120 | 240 | 180 | 20 | 120[a] | 40 | 40 | 120 | 120 | 10 | 90[a] | 150 |
| 8 | 40 | 150 | 240 | 30 | 120 | 120 | 30 | 90 | 90[a] | 30 | 120 | 120 |
| 9 | 90 | 150 | 150 | 40 | 40 | 40 | 90 | 120 | 120 | 30 | 90 | 90 |
| 10 | 120 | 120 | 120 | 40 | 120 | 120[a] | 30 | 40 | 90 | 20 | 90 | 90 |
| 11 | 60 | 120 | 120 | 40 | 60 | 60 | 40 | 90 | 90 | 20 | 60 | 30 |
| 12 | 120 | 240 | 240 | 20 | 90 | 90 | 30 | 90 | 90 | 30 | 180 | 60 |
| 13 | 90 | 150 | 150 | 30 | 90 | 90 | 30 | 90 | 90 | 20 | 40 | 60 |
| 14 | 150 | 150 | 150[a] | 30 | 40 | 90 | 20 | 90 | 90 | 30 | 90[a] | 90[a] |
| 15 | 120 | 180 | 180 | 30 | 60[a] | 120 | 30 | 120 | 120 | 30 | 90[a] | 90[a] |
| 16 | 90 | 180 | 180 | 40 | 90 | 120 | 20 | 90 | 60 | 30 | 150 | 120 |
| Mean | 102 | 180 | 186 | 31 | 85 | 104 | 36 | 87 | 93.8 | 25 | 91.3 | 93.8 |
| S.D. | 47.9 | 51.4 | 46.8 | 6.8 | 32.5 | 47.0 | 17.1 | 22.7 | 21.6 | 7.3 | 37.6 | 28.7 |

Treatment A - 400-mg tablet CLINORIL.
Treatment B - Tablet containing 424 mg sodium sulindac and 296 mg tromethamine.
Treatment C - Tablet containing 424 mg sodium sulindac and 660 mg tromethamine.
Treatment D - Solution containing 424 mg sodium sulindac and 660 mg tromethamine.
[a] - Evidence of enterohepatic recycling - earliest $t_{max}$ value chosen.

Table 4. Differences in Time (Hours to $t_{max}$ for Sulindac and Sulindac Sulfide for Tromethamine Formulations Compared With a CLINORIL Tablet

| Subject | Treatment B Sulindac | Treatment B Sulfide | Treatment C Sulindac | Treatment C Sulfide | Treatment D Sulindac | Treatment D Sulfide |
|---|---|---|---|---|---|---|
| 1 | 0.5 | 2.5 | 0.5 | 2.5 | 0.7 | 2.5 |
| 2 | 0.5 | 0 | 0.3 | 0.5 | 0.5 | 1.0 |
| 3 | 0.7 | -1.5 | 0.5 | 1.5 | 0.7 | 1.0 |
| 4 | 1.0 | 2.5 | 0.5 | 2.0 | 0.8 | 2.0 |
| 5 | 3.5 | 2.0 | 3.5 | 2.5 | 3.7 | 2.5 |
| 6 | 1.5 | 2.5 | 1.5 | 3.0 | 1.7 | 2.0 |
| 7 | 1.7 | 2.3 | 1.3 | 1.0 | 1.8 | 0.5 |
| 8 | 0.2 | 2.0 | 0.2 | 0 | 0.2 | 2.0 |
| 9 | 0.8 | 1.8 | 2.5 | 0.5 | 1.0 | 1.0 |
| 10 | 1.3 | 0 | 1.5 | 0.5 | 1.7 | 0.5 |
| 11 | 0.3 | 1.0 | 0.3 | 0.5 | 0.7 | 1.5 |
| 12 | 1.7 | 2.5 | 1.5 | 2.5 | 1.5 | 3.0 |
| 13 | 1 | 1.0 | 1.0 | 1.0 | 1.2 | 1.5 |
| 14 | 2 | 1.0 | 2.2 | 1.0 | 2.0 | 1.0 |
| 15 | 1.5 | 1.0 | 1.5 | 1.0 | 1.5 | 1.5 |
| 16 | 0.8 | 1.0 | 1.2 | 2.0 | 1.0 | 1.0 |
| Mean | 1.2 | 1.4 | 1.3 | 1.4 | 1.3 | 1.5 |
| S.D. | 0.82 | 1.1 | 0.9 | 0.9 | 0.8 | 0.7 |

Treatment B - Tablet containing 424 mg sodium sulindac and 296 mg tromethamine.
Treatment C - Tablet containing 424 mg sodium sulindac and 660 mg tromethamine.
Treatment D - Solution containing 424 mg sodium sulindac and 660 mg tromethamine.

Table 5. Summary of Kinetic Data

**Plasma**

| | | Treatment A* | Treatment B | Treatment C | Treatment D |
|---|---|---|---|---|---|
| $T_{max}$ (min.) | Sulindac | 102 ± 47.9 | 31 ± 6.8 | 36.1 ± 17.1 | 25 ± 7.3 |
| | Sulfone | 180 ± 51.4 | 85 ± 32.5 | 87 ± 22.7 | 91.3 ± 37.6 |
| | Sulfide | 186 ± 46.8 | 104 ± 47.0 | 93.8 ± 21.6 | 93.8 ± 28.7 |
| $\Delta T_{max}$ (hr) | Sulindac | - | 1.2 ± 0.8 | 1.3 ± 0.9 | 1.3 ± 0.8 |
| | Sulfide | - | 1.4 ± 1.1 | 1.4 ± 0.9 | 1.5 ± 0.7 |
| $C_{max}$ (μg/ml) | Sulindac | 9.6 ± 5.7 | 19.8 ± 10.6 | 21.6 ± 8.0 | 20.2 ± 6.9 |
| | Sulfone | 2.3 ± 0.8 | 2.7 ± 1.6 | 3.1 ± 3.7 | 2.4 ± 0.6 |
| | Sulfide | 5.2 ± 2.6 | 6.1 ± 2.3 | 6.2 ± 2.5 | 6.5 ± 2.3 |

**Urine**

| | Treatment A | Treatment B | Treatment C | Treatment D |
|---|---|---|---|---|
| Recovery of sulindac (mg) | 62.6 ± 38.2 | 61.9 ± 21.2 | 62.6 ± 21.8 | 61.8 ± 20.1 |
| Recovery of trans-sulindac (mg) | 4.9 ± 4.0 | 6.0 ± 4.0 | 6.4 ± 5.9 | 3.9 ± 3.8 |
| Recovery of sulfone (mg) | 53.4 ± 14.6 | 50.5 ± 14.6 | 47.8 ± 15.4 | 55.4 ± 29.3 |
| Recovery of sulfide (mg) | 0.3 ± 0.7 | 0.6 ± 1.7 | 0.2 ± 0.5 | 0.5 ± 1.6 |
| Total recovery of sulindac (mg) | 118.9 ± 51.9 | 116.9 ± 28.3 | 114.8 ± 32.4 | 119.2 ± 43.3 |
| % Recovery (geometric mean) | 27.9 | 28.4 | 27.4 | 28.4 |

**All values are mean ± S.D. for 18 subjects.**

***Treatment A - 400-mg tablet CLINORIL.**
**Treatment B - Tablet containing 424 mg sodium sulindac and 296 mg tromethamine.**
**Treatment C - Tablet containing 424 mg sodium sulindac and 660 mg tromethamine.**
**Treatment D - Solution containing 424 mg sodium sulindac and 660 mg tromethamine.**

In the presence of tromethamine, both peak plasma levels and peak time of sulindac following oral administration were improved, while urinary recovery of sulindac remained unchanged. These results show that the time to peak bioavailability of the sulindac sodium/tromethamine admixture is from I.2 hours to I.4 hours shorter than that of sulindac alone, so that an earlier clinical onset with the sulindac sodium/tromethamine admixture is predicted.

## Claims

I. A combination, comprising: (a) sodium sulindac or sulindac; and (b) tromethamine; or a pharmaceutically acceptable formulation thereof.

2. The combination of Claim I, further comprising: glyceryl behenate.

3. The combination of Claim 2, further comprising: starch.

4. The combination of Claim 3, further comprising: docusate sodium.

5. The combination of Claim 4 comprising (a) 2l2 mg to 424 mg of sodium sulindac or 200 to 400 mg of sulindac; (b) 295 to 300 mg of tromethamine; (c) 30 mg of glyceryl behenate; (d) 40 mg of corn starch; and (e) l.5 mg of docusate sodium.

6. A pharmaceutical composition for the treatment of inflammation and pain comprising a therapeutically sufficient amount of the combination of Claim l and a pharmaceutically acceptable carrier.

7. The pharmaceutical composition of Claim 6 comprising (a) 2l2 mg to 424 mg of sodium sulindac or 200 mg to 400 mg of sulindac; (b) 295 mg to 300 mg of tromethamine; (c) 30 mg of glycerly behante; (d) 40 mg of starch; (e) l.5 mg of docusate sodium and a pharmaceutically acceptable carrier.

8. The use of the combination of Claim 1 for the preparation of a medicament useful for the treatment of inflammation and pain.

9. The use as claimed in Claim 8 wherein the combination comprises (a) 212 mg to 424 mg of sodium sulindac or 200 mg to 400 mg of sulindac; (b) 295 mg to 300 mg of tromethamine; (c) 30 mg of glyceryl behenate; (d) 40 mg of starch; and (e) 1.5 mg of docusate sodium.

10. A combination comprising (a) sodium sulindac, and (b) sodium bicarbonate.

Claims for the following Contracting States : AT, GR, ES

1. A process for preparing a combination, which comprises mixing: (a) sodium sulindac or sulindac; and (b) tromethamine, and optionally producing a pharmaceutically acceptable formulation thereof.

2. The process of Claim 1, further comprising adding: glyceryl behenate.

3. The process of Claim 2, further comprising adding: starch.

4. The process of Claim 3, further comprising adding: docusate sodium.

5. The process of Claim 4 comprising mixing (a) 212 mg to 424 mg of sodium sulindac or 200 to 400 mg of sulindac; (b) 295 to 300 mg of tromethamine, (c) 30 mg of glyceryl behenate; (d) 40 mg of corn starch; and (e) 1.5 mg of docusate sodium.

6. A process for preparing à pharmaceutical composition for the treatment of inflammation and pain which comprises mixing a therapeutically sufficient amount of the combination prepared according to Claim 1 and a pharmaceutically acceptable carrier.

7. The process of Claim 6 which comprises mixing (a) 212 mg to 424 mg of sodium sulindac or 200 mg to 400 mg of sulindac; (b) 295 mg to 300 mg of tromethamine; (c) 30 mg of glycerly behante; (d) 40 mg of starch; (e) 1.5 mg of docusate sodium and a pharmaceutically acceptable carrier.

8. A process for preparing a combination which comprises mixing (a) sodium sulindac, and (b) bicarbonate.

European Patent Office

# EUROPEAN SEARCH REPORT

Application Number

EP 87 30 7295

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| X | US-A-4 307 114 (D.M. DVORNIK)<br>* Column 1, line 60 - column 2, line 48 *<br>--- | 1-10 | A 61 K 33/00<br>A 61 K 31/19 //<br>(A 61 K 33/00<br>A 61 K 31:19 )<br>(A 61 K 31/19<br>A 61 K 31:13 ) |
| D,A | US-A-3 654 349 (TSUNG-YING SHEN)<br>--- | 1-10 | |
| D,A | GB-A-2 059 768 (ILONA KAHAN)<br>----- | 1-10 | |
| | | | TECHNICAL FIELDS SEARCHED (Int. Cl.4) |
| | | | A 61 K |

The present search report has been drawn up for all claims

| Place of search | Date of completion of the search | Examiner |
| --- | --- | --- |
| THE HAGUE | 02-12-1987 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)